# FASCICULE DE BREVET EUROPEEN

(11) **EP 2 642 936 B1**
(45) Date de publication et mention de la délivrance du brevet: **20.05.2020**
(21) Numéro de dépôt: 11794545.1
(22) Date de dépôt: 10.11.2011
(51) Int. Cl.: A61B 17/72

(54) **CLOU D'OSTÉOSYNTHÈSE**
OSTEOSYNTHESESTIFT
OSTEOSYNTHESIS PIN

(30) Priorité: 25.11.2010 FR 1059724
(43) Date de publication de la demande: 02.10.2013
(73) Titulaire: Fournitures Hospitalieres Industrie, 29000 Quimper (FR); Goldzak, Mario, 31130 Balma (FR); Simon, Patrick, 69002 Lyon (FR); Mittlmeier, Thomas, 14193 Berlin (DE)
(72) Inventeur: GOLDZAK, Mario, F-31130 Balma (FR); SIMON, Patrick, F-69002 Lyon (FR); MITTLMEIER, Thomas, D-14193 Berlin (DE); HUNT, Franck, F-29000 Quimper (FR)
(74) Mandataire: Verriest, Philippe
(86) Numéro de dépôt international: PCT/FR2011/052618
(87) Numéro de publication internationale: WO 2012/069727

(56) Documents cités:
- EP-A1- 0 922 437
- WO-A1-2006/091460
- WO-A1-2007/125497
- WO-A2-2010/082183
- US-A1- 2007 288 016
- US-A1- 2010 114 315
- US-B1- 6 558 388

## Description

La présente invention concerne un clou d'ostéosynthèse, et plus particulièrement un clou d'ostéosynthèse pour fracture d'un calcanéum.

Afin de consolider une fracture d'un os, et plus particulièrement d'un calcanéum, il existe différentes techniques opératoires.

Une première technique connue consiste à immobiliser le pied d'un patient dans un plâtre. Une telle technique évite tout risque d'infection du patient, mais ne peut être utilisée que pour des fractures simples, c'est-à-dire sans déplacement de fragments osseux fracturés.

Les fractures complexes, c'est-à-dire avec déplacement de fragments osseux fracturés, doivent être traitées chirurgicalement en utilisant différents moyens d'ostéosynthèse.

Une première technique chirurgicale consiste à insérer des vis d'ostéosynthèse classiques ou des vis d'ostéosynthèse dites « de compression » dans les fragments osseux fracturés de manière à solidariser ces derniers à la partie principale de l'os afin de permettre une consolidation osseuse naturelle du foyer de fracture. De telles vis peuvent être posées en percutanée, et de ce fait ne nécessitent pas la réalisation d'incision cutanée de taille importante. Cependant, afin d'assurer une stabilisation satisfaisante des fragments osseux fracturés, il est souvent nécessaire de prévoir la pose de plusieurs vis d'ostéosynthèse par fragment osseux fracturés.

Une seconde technique chirurgicale consiste à implanter dans le pied du patient une plaque d'ostéosynthèse par abord latéral externe. L'implantation d'une telle plaque d'ostéosynthèse permet d'assurer une stabilisation satisfaisante des fragments osseux fracturés, mais conduit souvent à des complications cutanées, voire à des nécroses. De plus, l'implantation d'une telle plaque d'ostéosynthèse nécessite une incision cutanée de taille importante, ce qui augmente les risques d'infection du patient.

La présente invention vise à remédier à ces inconvénients.

Le document US 6,558,388 B1 montre un clou d'ostéosynthèse, constitué d'un corps creux de forme générale cylindrique comportant un alésage axial s'étendant sur toute sa longueur, une pluralité de trous traversants décalés longitudinalement les uns par rapport aux autres et destinés au passage de vis de fixation, et au moins une lumière oblongue.

Le problème technique à la base de l'invention consiste donc à fournir un clou d'ostéosynthèse qui soit de structure simple et économique, tout en limitant les risques d'infection du patient et favorisant la consolidation osseuse.

A cet effet, la présente invention concerne un clou d'ostéosynthèse tel que défini dans la revendication 1, notamment pour la fracture d'un calcanéum, constitué par un corps creux de forme générale cylindrique comportant un alésage axial s'étendant sur toute sa longueur, une pluralité de trous traversants décalés longitudinalement les uns par rapport aux autres et destinés au passage de vis de fixation, et au moins une lumière oblongue adaptée pour le passage d'un ancillaire destiné au repositionnement de fragments osseux fracturés.

La structure du clou selon l'invention permet d'insérer ce dernier dans l'os du patient en pratiquant une faible incision cutanée, et de ce fait limite les risques d'infection du patient.

De plus, la présence des trous traversants ménagés dans le corps du clou permet d'assembler de manière stable, à l'aide de vis d'ostéosynthèse, les fragments osseux fracturés à la partie principale de l'os dans laquelle est implanté le clou.

La présence de l'alésage axial et de l'au moins une lumière oblongue ménagés dans le corps permet notamment l'insertion d'un ancillaire courbe, tel qu'un chasse-greffon, ou d'une broche courbe à l'intérieur du corps depuis l'extérieur du pied et le passage de l'extrémité libre de celui-ci ou de celle-ci à travers la lumière afin de coopérer avec les fragments fracturés pour les remettre en place. La présence de l'alésage axial et de l'au moins une lumière oblongue permet également le passage d'une caméra d'arthroscopie afin de vérifier cette remise en place des fragments fracturés.

En outre, le fait que le corps comporte un alésage axial s'étendant sur toute la longueur de ce dernier permet de repositionner la carotte osseuse obtenue lors de la préparation de l'os destiné à recevoir le clou, c'est-à-dire lors de la réalisation du forage osseux destiné à recevoir le clou, à l'intérieur du corps qui agit alors comme une cage d'arthrodèse. Ces dispositions permettent d'améliorer encore la consolidation osseuse, et également de diminuer la convalescence du patient.

Le corps comporte deux lumières oblongues diamétralement opposées.

Chaque lumière s'étend selon une longueur correspondant sensiblement à au moins 40% de la longueur du clou.

De façon avantageuse, chaque lumière s'étend sensiblement parallèlement à l'axe du clou.

De façon préférentielle, chaque trou traversant présente un axe s'étendant sensiblement perpendiculairement à l'axe du clou, et les axes des trous traversants sont sensiblement parallèles.

De façon avantageuse, chaque lumière est orientée selon un angle compris entre 80 et 100° par rapport aux axes des trous traversants du clou, et de préférence d'environ 90°.

De préférence, l'extrémité distale du corps comprend au moins une dent agencée pour coopérer avec l'os lors de l'insertion du clou dans celui-ci.

Le corps comporte avantageusement des moyens d'indexage angulaire d'un ancillaire conçu pour la pose dudit clou, les moyens d'indexage étant de préférence disposés au niveau de l'extrémité proximale du clou. Les moyens d'indexage comportent par exemple une rainure d'indexage débouchant dans l'extrémité proximale du clou et d'axe sensiblement parallèle à l'axe des trous traversants.

Selon un mode de réalisation, le corps comprend des moyens de fixation destinés à coopérer avec des moyens de fixation complémentaires portés par un ancillaire conçu pour la pose dudit clou. L'alésage axial comprend par exemple une portion filetée formant les moyens de fixation. La portion filetée est avantageusement disposée à proximité de l'extrémité proximale du corps. De préférence, le diamètre interne du corps au niveau des sommets des filets correspond sensiblement au diamètre interne du corps en dehors de la portion filetée.

Avantageusement, les trous traversants sont décalés longitudinalement de manière équidistante. Les trous traversants sont adaptés par exemple pour le passage de vis de fixation de diamètre compris entre 4 et 6 mm. Le corps présente avantageusement entre deux et quatre trous traversants.

Le corps est de préférence réalisé en matériau biocompatible, tel qu'en acier inoxydable ou en alliage de titane. Le corps présente une longueur comprise par exemple entre 30 et 70 mm, et un diamètre externe compris par exemple entre 8 et 15 mm.

Le corps présente avantageusement un diamètre interne sensiblement constant sur toute sa longueur

De toute façon l'invention sera bien comprise à l'aide de la description qui suit en référence au dessin schématique annexé représentant, à titre d'exemples non limitatifs, plusieurs formes d'exécution de ce clou d'ostéosynthèse.
Figure 1 est une vue en perspective d'un clou d'ostéosynthèse selon l'invention.
Figure 2 est une vue arrière en perspective du clou de la figure 1.
Figures 3 et 4 sont des vues en perspective du clou de la figure 1 monté sur un ancillaire adapté pour la pose dudit clou.

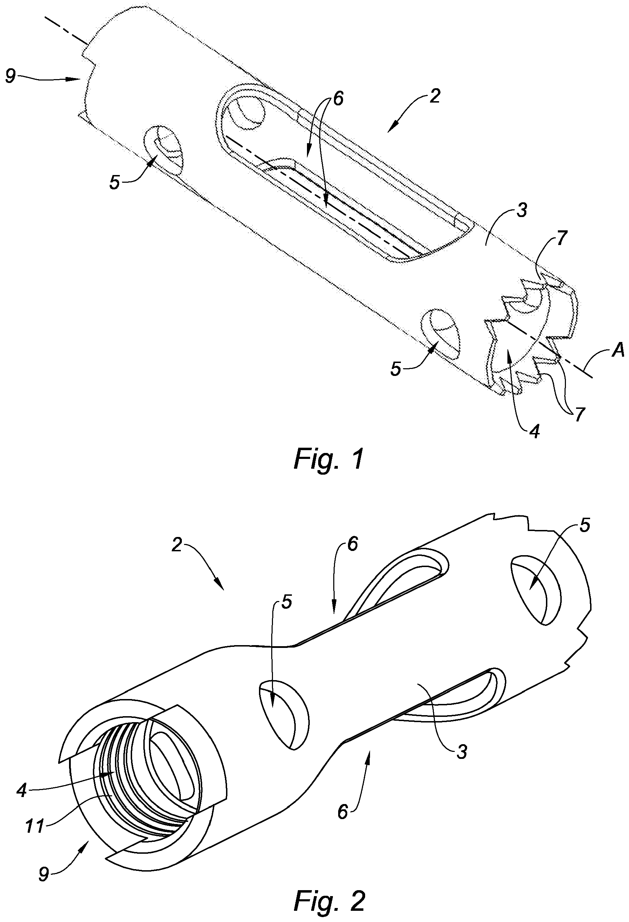

Les figures 1 et 2 représentent un clou d'ostéosynthèse 2 pour la fracture d'un calcanéum.

Le clou d'ostéosynthèse 2 comprend un corps creux 3 de forme générale cylindrique. Le corps 3 présente une longueur comprise par exemple entre 30 et 70 mm, et est réalisé en matériau biocompatible, tel qu'en acier inoxydable ou en alliage de titane. Le corps présente avantageusement un diamètre externe compris entre 8 et 15 mm.

Le corps creux 3 comporte un alésage axial 4 s'étendant sur toute sa longueur, et de section sensiblement circulaire.

Le corps creux 3 comporte de plus une pluralité de trous traversants 5 décalés longitudinalement les uns par rapport aux autres et destinés au passage de vis de fixation. Les axes des trous traversants 5 sont sensiblement parallèles entre eux et s'étendant sensiblement perpendiculairement à l'axe A du clou.

Le corps 3 présente avantageusement entre deux et quatre trous traversants 5. Les trous traversants 5 sont de préférence adaptés pour le passage de vis de fixation de diamètre compris entre 4 et 6 mm. Avantageusement, lorsque le corps creux 3 comporte au moins trois trous traversants 5, ces derniers sont décalés longitudinalement de manière équidistante.

Le corps creux 3 comporte en outre deux lumières oblongues 6 diamétralement opposées adaptées chacune pour le passage d'un ancillaire destiné au repositionnement de fragments osseux fracturés, tel qu'un chasse-greffon courbe ou une broche courbe.

Chaque lumière oblongues 6 s'étend sensiblement parallèlement à l'axe A du clou, et présente une longueur correspondant sensiblement à au moins 40% de la longueur du clou.

Comme montrée plus particulièrement sur la figure 1, chaque lumière oblongue 6 est orientée selon un angle compris entre 80 et 100° par rapport aux axes des trous traversants 5 du clou, et de préférence d'environ 90°.

L'extrémité distale du corps 3, c'est-à-dire l'extrémité du corps destinée à être introduite en premier dans l'os, comprend une pluralité de dents 7 agencées pour coopérer avec l'os lors de l'insertion du clou 2 dans celui-ci.

Le corps 3 comporte également des moyens d'indexage angulaire d'un ancillaire 8 conçu pour la pose dudit clou. Les moyens d'indexage comportent avantageusement une rainure d'indexage 9 débouchant dans l'extrémité proximale du clou et d'axe sensiblement parallèle à l'axe des trous traversants 5.

Le corps 3 comporte en outre des moyens de fixation destinés à coopérer avec des moyens de fixation complémentaires portés par l'ancillaire conçu pour la pose dudit clou.

Comme montré sur la figure 2, l'alésage axial 4 comprend une portion filetée 10 disposée à proximité de l'extrémité proximale du corps, la portion filetée 10 formant les moyens de fixation. De préférence, le diamètre interne du corps 3 au niveau des sommets des filets de la portion filetée 10 correspond sensiblement au diamètre interne du corps en dehors de la portion filetée. Ainsi, le corps 3 présente un diamètre interne sensiblement constant sur toute sa longueur.

Selon une variante de réalisation de l'invention non représentée sur les figures, le corps 3 pourrait comporter quatre trous traversants 5 d'axes équidistants, et le nombre et le profil des dents 7 pourraient être différents.

Lors d'une opération d'ostéosynthèse visant à rattacher une pluralité de fragments osseux au corps du calcanéum correspondant, un chirurgien procède de la façon suivante.

Tout d'abord, le chirurgien réalise un forage dans le calcanéum, et plus particulièrement dans la grosse tubérosité calcanéenne, à l'aide d'une tarière présentant un diamètre interne correspondant sensiblement au diamètre interne du corps 3. Le forage est de préférence réalisé selon l'axe des travées.

Ensuite, le chirurgien introduit le clou 2 à l'intérieur du forage osseux à l'aide d'un ancillaire adapté. Avantageusement, le clou 2 est entraîné en rotation à l'aide de cet ancillaire lors de son insertion dans le forage, de telle sorte que les dents 7 du clou coopèrent avec le calcanéum de manière à agrandir légèrement le diamètre du forage osseux. Il convient de noter que le clou 2 est positionné dans le forage osseux de telle sorte que les axes des trous traversants 5 s'étendent dans un plan sensiblement parallèle à la face inférieure du pied.

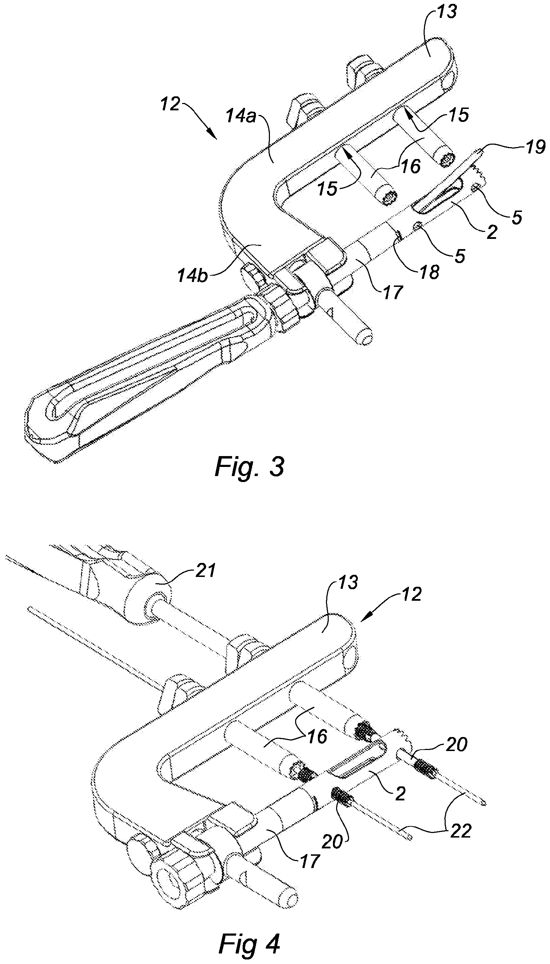

Puis, le chirurgien fixe un ancillaire 12, tel que représenté sur les figures 3 et 4, sur le clou 2 préalablement implanté dans le calcanéum.

L'ancillaire 12 comporte une portion de guidage 13 en forme générale de L présentant une première branche 14a comportant une pluralité d'orifices traversants 15 décalés les uns par rapport aux autres et destinés aux passages de canon de perçage 16, et une deuxième branche 14b perpendiculaire à la première branche. L'ancillaire 12 comprend en outre une portion cylindrique 17 d'axe parallèle à la première branche 14a de la portion de guidage 13, la portion cylindrique 17 comprenant deux doigts d'indexage angulaire 18 diamétralement opposés agencés pour coopérer avec la rainure d'indexage 9 ménagée dans la portion proximale du clou 2.

L'ancillaire 12 comprend également des moyens d'immobilisation conçus pour permettre une immobilisation de l'ancillaire sur le clou 2. Les moyens d'immobilisation comportent avantageusement une vis de fixation (non représentée sur les figures) s'étendant à travers la portion cylindrique 17 et agencée pour coopérer avec la portion filetée 10 ménagée au niveau de l'extrémité proximale du clou.

Le chirurgien introduit ensuite un ancillaire courbe 19 dans l'alésage axial 4 du corps 3 depuis l'extérieur du pied en passant par la portion cylindrique 17 et l'extrémité proximale du corps, et fait passer l'extrémité libre de cet ancillaire 19 à travers l'une des lumières 6 afin de déplacer les fragments osseux fracturés nécessitant un repositionnement. Un tel repositionnement des fragments osseux fracturés peut éventuellement être contrôlé en introduisant une caméra d'arthrodèse à l'intérieur de l'alésage axial 4 du clou 2.

Il convient de noter que l'axe de la portion cylindrique 17 est sensiblement confondu avec l'axe A du clou en position immobilisée de l'ancillaire 12 sur ledit clou, et que l'ancillaire 12 est conçu et les différents orifices 15 sont positionnés de telle sorte qu'en position immobilisée de l'ancillaire sur le clou 2, l'axe de chaque orifice 15 ménagé sur la portion de guidage 13 est confondu avec l'axe d'un trou traversant 5 ménagé dans le corps dudit clou.

Le chirurgien réalise ensuite des alésages osseux en regard des trous traversants 5 du clou 2 dans lesquels sont destinées à être introduites des vis d'ostéosynthèse pour l'assemblage des fragments fracturés à la partie principale du calcanéum. Ces alésages sont réalisés à l'aide d'un ancillaire adapté, tel qu'une perceuse, dont l'organe de perçage est destiné à coulisser le long des canons de perçage 16.

Enfin, le chirurgien introduit des vis d'ostéosynthèse 20 dans les alésages osseux précédemment réalisés et dans les trous traversants 5 correspondants à l'aide de l'ancillaire adapté, tel qu'un tournevis 21.

Il doit être noté que le perçage des alésages osseux et le vissage des vis d'ostéosynthèse peuvent être guidés à l'aide de broches de guidage 22 préalablement implantées dans les fragments osseux fracturés ou dans la partie principale du calcanéum.

Comme il va de soi, l'invention ne se limite pas aux seules formes d'exécution de ce clou d'ostéosynthèse, décrites ci-dessus à titre d'exemples, elle en embrasse au contraire toutes les variantes de réalisation.

## Revendications

1. Clou d'ostéosynthèse (2), notamment pour fracture d'un calcanéum, constitué d'un corps creux (3) de forme générale cylindrique comportant :
- un alésage axial (4) s'étendant sur toute la longueur du corps creux (3) et adapté à recevoir une carotte osseuse,
- une pluralité de trous traversants (5) décalés longitudinalement les uns par rapport aux autres et destinés au passage de vis de fixation, et
- deux lumières oblongues (6) diamétralement opposées et adaptées chacune pour le passage d'un ancillaire (19) destiné au repositionnement de fragments osseux fracturés, chaque lumière (6) s'étendant selon une longueur correspondant sensiblement à au moins 40% de la longueur du clou (2),
le corps (3) présentant un diamètre interne sensiblement constant sur toute sa longueur.

2. Clou d'ostéosynthèse selon la revendication 1, **caractérisé en ce que** chaque lumière (6) s'étend sensiblement parallèlement à l'axe (A) du clou.

3. Clou d'ostéosynthèse selon la revendication 1 ou 2, **caractérisé en ce que** chaque trou traversant (5) présente un axe s'étendant sensiblement perpendiculairement à l'axe (A) du clou, et **en ce que** les axes des trous traversants (5) sont sensiblement parallèles.

4. Clou d'ostéosynthèse selon la revendication 3, **caractérisé en ce que** chaque lumière (6) est orientée selon un angle compris entre 80 et 100° par rapport aux axes des trous traversants du clou, et de préférence d'environ 90°.

5. Clou d'ostéosynthèse selon l'une des revendications 1 à 4, **caractérisé en ce que** l'extrémité distale du corps (3) comprend au moins une dent (7) agencée pour coopérer avec l'os lors de l'insertion du clou dans celui-ci.

6. Clou d'ostéosynthèse selon l'une des revendications 1 à 5, **caractérisé en ce que** le corps (3) comporte des moyens d'indexage angulaire d'un ancillaire conçu pour la pose dudit clou, les moyens d'indexage étant de préférence disposés au niveau de l'extrémité proximale du clou.

7. Clou d'ostéosynthèse selon la revendication 6, **caractérisé en ce que** les moyens d'indexage comportent une rainure d'indexage (9) débouchant dans l'extrémité proximale du clou et d'axe sensiblement parallèle à l'axe des trous traversants (5).

8. Clou d'ostéosynthèse selon l'une des revendications 1 à 7, **caractérisé en ce que** le corps (3) comprend des moyens de fixation destinés à coopérer avec des moyens de fixation complémentaires portés par un ancillaire conçu pour la pose dudit clou.

## Patentansprüche

1. Osteosynthesestift (2), insbesondere für die Fraktur eines Fersenbeins, bestehend aus einem Hohlkörper (3) in einer im Allgemeinen zylindrischen Form, Folgendes beinhaltend:
- eine axiale Ausbohrung (4), die sich über die gesamte Länge des Hohlkörpers (3) erstreckt, und ausgeführt ist, um einen Knochenzylinder aufzunehmen,
- eine Vielzahl von Durchgangslöchern (5), die in Längsrichtung in Bezug zueinander versetzt sind, und für den Durchgang einer Befestigungsschraube bestimmt sind, und
- zwei Langlöcher (6), die diametral entgegengesetzt sind, und jeweils für den Durchgang eines Hilfsinstruments (19) ausgeführt sind, das zur Neupositionierung der frakturierten Knochenfragmente bestimmt ist, wobei sich jedes Langloch (6) in eine Länge erstreckt, die im Wesentlichen mindestens 40 % der Länge des Stifts (2) entspricht,
wobei der Körper (3) einen im Wesentlichen über seine gesamte Länge konstanten Innendurchmesser aufweist.

2. Osteosynthesestift nach Anspruch 1, **dadurch gekennzeichnet, dass** sich jedes Langloch (6) im Wesentlichen parallel zur Achse (A) des Stiftes erstreckt.

3. Osteosynthesestift nach Anspruch 1 oder 2, **dadurch gekennzeichnet, dass** jedes Durchgangsloch (5) eine Achse aufweist, die sich im Wesentlichen senkrecht zur Achse (A) des Stifts erstreckt, und dadurch, dass die Achsen der Durchgangslöcher (5) im Wesentlichen parallel sind.

4. Osteosynthesestift nach Anspruch 3, **dadurch gekennzeichnet, dass** jedes Langloch (6) in einem Winkel ausgerichtet ist, der zwischen 80 und 100° in Bezug auf die Achsen der Durchgangslöcher des Stiftes enthalten ist, und vorzugsweise etwa 90° beträgt.

5. Osteosynthesestift nach einem der Ansprüche 1 bis 4, **dadurch gekennzeichnet, dass** das distale Ende des Körpers (3) mindestens einen Zahn (7) umfasst, der angeordnet ist, um beim Einführen des Stifts in denselben mit dem Knochen zusammenzuwirken.

6. Osteosynthesestift nach einem der Ansprüche 1 bis 5, **dadurch gekennzeichnet, dass** der Körper (3) Winkelindexiermittel für ein Hilfsmittel beinhaltet, das zum Einsetzen des Stiftes gestaltet ist, wobei die Indexiermittel vorzugsweise im Bereich des proximalen Endes des Stiftes angeordnet sind.

7. Osteosynthesestift nach Anspruch 6, **dadurch gekennzeichnet, dass** die Indexiermittel eine Indexiernut (9) beinhalten, die in das proximale Ende des Stiftes mündet, und mit einer zur Achse der Durchgangslöcher (5) im Wesentlichen parallelen Achse.

8. Osteosynthesestift nach einem der Ansprüche 1 bis 7, **dadurch gekennzeichnet, dass** der Körper (3) Befestigungsmittel umfasst, die dazu bestimmt sind, mit ergänzenden Befestigungsmitteln zusammenzuwirken, die von einem Hilfsmittel getragen werden, das zum Einsetzen des Stiftes gestaltet ist.

## Claims

1. An osteosynthesis pin (2), in particular for the fracture of a calcaneum, made up of a generally cylindrical hollow body (3) comprising:
- an axial bore (4) extending over the entire length of the body (3) and adapted to receive a bone cylinder,
- a plurality of through-holes (5) staggered longitudinally relative to one another and intended for attachment screws to pass therethrough, and
- two diametrically opposite oblong openings (6) suitable for an ancillary instrument to pass therethrough for repositioning fractured bone fragments, each opening (6) extends over a length corresponding substantially to at least 40% of the length of the osteosynthesis pin (2),
the body having a substantially constant inner diameter over its entire length.

2. The osteosynthesis pin according to claim 1, **characterized in that** each opening (6) extends substantially parallel to the axis (a) of the pin.

3. The osteosynthesis pin according to claim 1 or 2, **characterized in that** each through-hole (5) has an axis extending substantially perpendicular to the axis (A) of the pin, and **in that** the axes of the through-holes (5) are substantially parallel.

4. The osteosynthesis pin according to claim 3, **characterized in that** each opening (6) is oriented at an angle comprised between 80 and 100° relative to the axes of the through-holes of the pin, and preferably approximately 90°.

5. The osteosynthesis pin according to any one of claims 1 to 4, **characterized in that** the distal end of the body (3) comprises at least one tooth (7) arranged to cooperate with the bone during the insertion of the pin therein.

6. The osteosynthesis pin according to any one of claims 1 to 5, **characterized in that** the body (3) includes angular indexing means for angularly indexing an ancillary instrument designed to implant said pin, the indexing means preferably being positioned at the proximal end of the pin.

7. The osteosynthesis pin according to claim 6, **characterized in that** the angular indexing means include an indexing slot (9) emerging in the proximal end of the pin and with an axis substantially parallel to the axis of the through-holes (5).

8. The osteosynthesis pin according to any one of claims 1 to 7, **characterized in that** the body (3) comprises fastening means designed to cooperate with complementary fastening means provided on an ancillary instrument designed to implant said pin.
